# EUROPEAN PATENT SPECIFICATION

(11) **EP 3 376 860 B1**
(45) Date of publication and mention of the grant of the patent: **12.07.2023**
(21) Application number: 16808832.6
(22) Date of filing: 03.11.2016
(51) Int. Cl.: A01N 1/02, C12M 1/00, B01L 3/00, B65D 83/00

(54) **CRYOPRESERVATION DEVICES**
KRYOKONSERVIERUNGSVORRICHTUNGEN
DISPOSITIFS DE CRYOCONSERVATION

(30) Priority: 16.11.2015 US 201562255627 P
(43) Date of publication of application: 26.09.2018
(73) Proprietor: Corning Incorporated, Corning, New York 14831 (US)
(72) Inventor: CODDAIRE, John Wilfred, Kennebunk, Maine 04043 (US); LACEY, William Joseph, North Andover, Massachusetts 01845 (US); MARTIN, Gregory Roger, Acton, ME 04001 (US); SCHAEFER, Michael Kurt, Gorham, Maine 04038 (US); SZLOSEK, Paul Michael, Kennebunk, Maine 04043 (US); TANNER, Allison Jean, Portsmouth, New Hampshire 03801 (US)
(74) Representative: Elkington and Fife LLP
(86) International application number: PCT/US2016/060299
(87) International publication number: WO 2017/087176

(56) References cited:
- EP-A1- 2 364 928
- EP-A1- 2 364 928
- CA-A1- 2 314 658
- CA-A1- 2 314 658
- CH-A- 240 683
- CH-A- 240 683
- US-A- 3 419 179
- US-A- 5 826 751
- US-A1- 2011 008 908
- US-A1- 2015 048 085

## Description

### FIELD OF THE DISCLOSURE

The present disclosure relates generally to cryopreseravation devices, and more specifically to cryogenic vial assemblies for preparing and storing frozen samples, and methods for removing frozen samples from such vial assemblies.

### BACKGROUND

Biological samples such as cells and tissues are often cryopreserved to extend their viability and usefulness for a variety of applications. For example, the cryopreservation process can involve placing a biological sample into an aqueous solution containing electrolytes and/or cryoprotectants and lowering the temperature of the solution to below its freezing point. Biological samples are often stored in vials which can be sealed and frozen, e.g., by immersion in liquid nitrogen. It can be important to maintain the sample integrity during the filling, storage, and retrieval stages, as contamination can render a biological sample useless for scientific research or other applications.

Vial leakage, which can be caused by a failure of the seal between the vial and the cap, can be a contributing factor to sample contamination. Sample contamination can also occur during thawing of the sample prior to its removal from the vial. For instance, cryogenic vials are often placed in a warm bath or heated block to partially or completely defrost the sample for ease of removal. However, samples can become contaminated or lose part of their viability during this process due to liquid immersion and/or elevated temperatures. The sample may also become overstressed due to excessive heating at the vial wall surface which can further damage the sample.

Removal of samples in the frozen state without thawing may reduce the risk of sample contamination and/or damage. However, it can be difficult to remove the frozen pellet from the vial due to adhesion of the sample to the vial walls and/or inability to grip and/or exert a force on the sample. Accordingly, it would be advantageous to provide vial assemblies from which frozen samples can be more easily discharged while also maintaining an acceptable sealed integrity for preventing sample contamination. It would also be advantageous to provide methods for preparing frozen samples which can be more easily discharged from a vial and methods for removing frozen samples from vials without the need for thawing prior to removal.
US 2015/048085 A1 relates to articles for cryopreservation.
US 3,419,179 A relates to a container for sample collection and laboratory processing.
US 2011/008908 A1 relates to an apparatus for separating and analyzing blood.
EP 2 364 928 A1 relates to a cylindrical shaped container, which has a body made of rigid material and a bottom made of flexible material, to allow deformation of the bottom under manual pressure.

### SUMMARY

The invention relates, in one embodiment, to vial assemblies comprising a tubular body comprising a cavity, a first end, a second end for dispensing a sample, and a tube wall comprising a first material; and a flexible bottom comprising a second elastomeric material overmolded on an interior surface of the tube wall proximate the first end. The flexible bottom does not extend past the first end of the tubular body. The tube wall comprises at least two flanges proximate the flexible bottom, wherein the flanges are configured to hinge inwardly upon application of force F to apply pressure to the flexible bottom. The invention relates, in another embodiment, to vial assemblies comprising a tubular body comprising a cavity, a first end, a second end, and a tube wall comprising a first material; and a flexible bottom comprising a second elastomeric material overmolded on the first end of the tubular body, wherein the flexible bottom further comprises at least two flanges attached to the tube wall and configured to hinge inwardly upon application of force F to apply pressure to a surface of the flexible bottom.

In yet another embodiment, the invention provides vial assemblies comprising a substantially rigid tubular body comprising a cavity, a first end, a second end, and a tube wall comprising a first material; and a flexible liner comprising a second elastomeric material positioned in the cavity of the tubular body, wherein the flexible liner further comprises at least two flanges, and wherein the flanges are configured to hinge inwardly upon application of force F to apply pressure to a surface of the flexible liner. Still further disclosed herein, but not forming part of the claimed invention, are vial assemblies comprising a tubular body comprising a cavity, a first end, a second end for dispensing a sample, a tube wall, and a flexible bottom disposed on an interior surface of the tube wall proximate the first end, wherein the tube wall and flexible bottom comprise a first material, wherein the flexible bottom has a first thickness less which is less than a second thickness of the tube wall, and wherein the flexible bottom does not extend past the first end of the tubular body.

Methods for preparing and/or storing frozen samples in a vial assembly are also disclosed herein, as well as methods for removing such samples from the vial assemblies. Such methods, which do not form part of the claimed invention, can comprise removing a frozen sample from a vial assembly comprising a tubular body having a first end and an overmolded flexible bottom proximate the first end by applying force to at least a portion of the overmolded flexible bottom, wherein the flexible bottom is configured to compress at least partially upon the application of force. Removing a frozen sample from a vial assembly comprising a tubular body and a flexible liner inserted into a cavity of the tubular body can also comprise depressing two or more flanges of the flexible liner to apply force to at least a portion of the flexible liner, wherein the flexible liner is configured to compress at least partially upon the application of force. Finally, removing a frozen sample from a vial assembly comprising a tubular body comprising a tube wall, a first end, and a flexible bottom proximate the first end can comprise applying force to at least a portion of the flexible bottom, wherein the flexible bottom is configured to compress at least partially upon the application of force, and wherein the flexible bottom has a first thickness which is less than a second thickness of the tube wall.

Vial assemblies disclosed herein may provide a user with the ability to loosen and/or remove a frozen sample from a vial without thawing the sample beforehand. Such vial assemblies may also provide enhanced grip for the user when handling the frozen sample. Methods for removing frozen samples from the vial assemblies disclosed herein may have improved consistency and/or repeatability, thereby saving the user time and/or decreasing variability from sample to sample due to varying sample removal conditions, e.g., differing time and/or temperature used to thaw a sample. It should be noted, however, that one or more of such benefits may not be present according to various embodiments of the disclosure, yet such embodiments are intended to fall within the scope of the disclosure.

The disclosure provides, in an aspect (1) a vial assembly comprising a tubular body comprising a cavity, a first end, a second end for dispensing a sample, and a tube wall comprising a first material; and a flexible bottom comprising a second elastomeric material overmolded on an interior surface of the tube wall proximate the first end, wherein the flexible bottom does not extend past the first end of the tubular body, and wherein the tube wall comprises at least two flanges proximate the flexible bottom, wherein the flanges are configured to hinge inwardly upon application of force F to apply pressure to the flexible bottom. One embodiment (2) provides the vial assembly of aspect 1, wherein the flanges further comprise at least one gusset configured to apply force to the flexible bottom.

In an aspect (3), the invention provides a vial assembly comprising: a tubular body comprising a cavity, a first end, a second end, and a tube wall comprising a first material; and a flexible bottom comprising a second elastomeric material overmolded on the first end of the tubular body, wherein the flexible bottom further comprises at least two flanges attached to the tube wall and configured to hinge inwardly upon application of force F to apply pressure to a surface of the flexible bottom.

In an aspect (4), the invention provides a vial assembly comprising: a substantially rigid tubular body comprising a cavity, a first end, a second end, and a tube wall comprising a first material; and a flexible liner comprising a second elastomeric material positioned in the cavity of the tubular body and extending past the first end of the substantially rigid tubular body, wherein the flexible liner further comprises at least two flanges, and wherein the flanges are configured to hinge inwardly upon application of force F to apply pressure to a surface of the flexible liner. One embodiment (5) provides the vial assembly of aspect 4, wherein the second end comprises a lip and wherein the flexible liner covers at least a portion of the lip. Another embodiment (6) provides the vial assembly of aspect 4 or 5 further comprising a cap, wherein the flexible liner is configured to form a seal between the lip and the cap in a closed position. Another embodiment (7) provides the vial assembly of any one of aspects 4-6 wherein the flexible liner is overmolded on an interior or exterior surface of the tube wall.

Additional features and advantages of the invention will be set forth in the detailed description which follows, and in part will be readily apparent to those skilled in the art from that description or recognized by practicing the invention as described herein, including the detailed description which follows, the claims, and the appended drawings.

It is to be understood that both the foregoing general description and the following detailed description present various embodiments of the disclosure, and are intended to provide an overview or framework for understanding the nature and character of the claims. The accompanying drawings are included to provide a further understanding of the disclosure, and are incorporated into and constitute a part of this specification. The drawings illustrate various embodiments of the disclosure and together with the description serve to explain the principles and operations of the invention.

### BRIEF DESCRIPTION OF THE DRAWINGS

The following detailed description can be best understood when read in conjunction with the following drawings, in which, where possible, like numbers are used to refer to like elements, and:
FIGS. 1A-B are cross-sectional views of a vial assembly according to aspects of the disclosure;
FIGS. 2A-C are cross-sectional views of a vial assembly according to various disclosed aspects of the disclosure;
FIGS. 3A-B are cross-sectional views of a vial assembly according to certain embodiments;
FIGS. 4A-B are cross-sectional views of a vial assembly according to additional disclosed embodiments;
**FIG. 5A** is a cross-sectional view of the bottom of a vial assembly according to certain aspects disclosed herein;
**FIG. 5B** is a perspective view of a vial assembly according to aspects disclosed herein;
**FIG. 6A** is a perspective view of a vial assembly according to further embodiments of the disclosure;
**FIG. 6B** is a cross-sectional view of a vial assembly according to various disclosed embodiments;
**FIG. 7A** is a perspective view of a vial assembly according to additional disclosed embodiments; and
**FIG. 7B** is a cross-sectional view of a vial assembly according to certain disclosed embodiments.

### DETAILED DESCRIPTION

### Vial Assemblies

Disclosed herein are vial assemblies comprising a tubular body comprising a cavity, a first end, a second, end for dispensing a sample, and a tube wall comprising a first material; and a flexible bottom comprising a second elastomeric material overmolded on an interior surface of the tube wall proximate the first end, wherein the flexible bottom does not extend past the first end of the tubular body. The tube wall comprises at least two flanges proximate the flexible bottom, and the flanges are configured to hinge inwardly upon application of force F to apply pressure to the flexible bottom. Also disclosed herein are vial assemblies comprising a tubular body comprising a cavity, a first end, a second end, and a tube wall comprising a first material; and a flexible bottom comprising a second elastomeric material overmolded on the first end of the tubular body, wherein the flexible bottom further comprises at least two flanges attached to the tube wall and configured to hinge inwardly upon application of force F to apply pressure to a surface of the flexible bottom.

Further disclosed herein are vial assemblies comprising a substantially rigid tubular body comprising a cavity, a first end, a second end, and a tube wall comprising a first material; and a flexible liner comprising a second elastomeric material positioned in the cavity of the tubular body and extending past the first end of the substantially rigid tubular body, wherein the flexible liner further comprises at least two flanges, and wherein the flanges are configured to hinge inwardly upon application of force F to apply pressure to a surface of the flexible liner. Still further disclosed herein, but not forming part of the claimed invention, are vial assemblies comprising a tubular body comprising a cavity, a first end, a second end for dispensing a sample, a tube wall, and a flexible bottom disposed on an interior surface of the tube wall proximate the first end, wherein the tube wall and flexible bottom comprise a first material, wherein the flexible bottom has a first thickness less which is less than a second thickness of the tube wall, and wherein the flexible bottom does not extend past the first end of the tubular body.

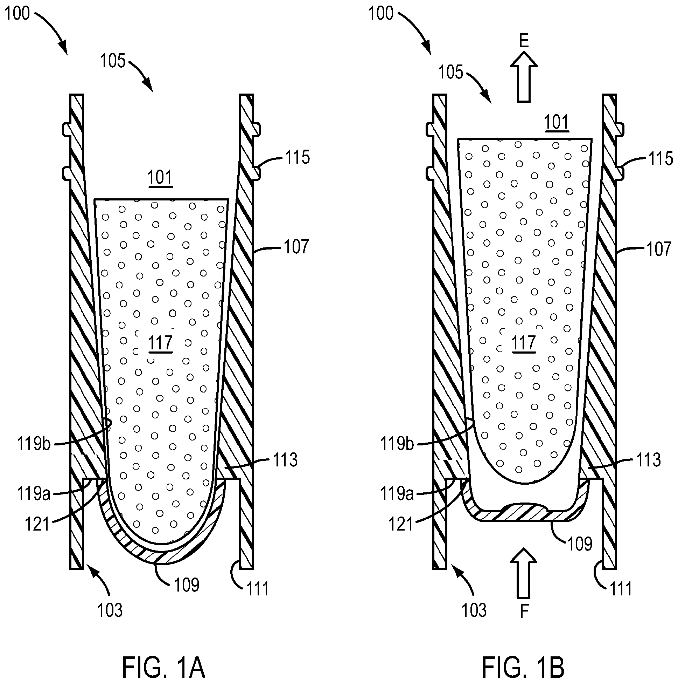

Embodiments and aspects of the disclosure will be discussed with reference to **FIGS. 1-7****,** which illustrate various non-limiting aspects of a vial assembly according to the disclosure. Of course, it is to be understood that the vial assemblies are not drawn to scale and the relative size and/or proportion of the entire assembly and/or of portions of the assembly relative to other portions of the assembly can be smaller or larger than those depicted. The following general description is intended to provide an overview of the claimed devices and various aspects will be more specifically discussed throughout the disclosure with reference to the non-limiting embodiments, these embodiments being interchangeable with one another within the context of the disclosure.

As demonstrated in FIGS. 1A-B, which are cross-sectional views of a vial assembly according to various aspects of the disclosure, a vial assembly can comprise a tubular body **100** including a cavity **101**, a first end **103**, a second end **105**, and a tube wall **107.** A flexible bottom **109** can be overmolded on an interior surface **111** of the tube wall **107** proximate the first end **103**. In some instances, and as illustrated, the flexible bottom **109** can be overmolded onto an interior feature **113** on the tube wall **107** interior surface **111**. In some aspects, threading **115** may be present on an exterior or interior surface of the tube wall **107** proximate the second end **105**, for securing a cap (not illustrated) thereto. As illustrated in **FIG. 1A**, the cavity **101** can contain a frozen sample or pellet **117**. As illustrated in **FIG. 1B**, the sample **117** can be at least partially dislodged by application of force **F** to the flexible bottom **109**, which can dislodge and/or eject the frozen sample in direction **E**, e.g., such that it can be dispensed via the second end **105**.

The tubular body **100** can comprise, for example, a substantially cylindrical or frusto-conical cavity **101**, which can be delineated by the tube wall **107** and the flexible bottom **109**. The tube wall **107** may be constructed from any material suitable for a vial assembly such as a cryotube. For example, the tube wall may be constructed from a first material chosen from polyolefins (e.g., polyethylene or polypropylene), fluoropolymers (e.g., polytetrafluoroethylene), polycarbonates, polyesters, polystyrenes, and other similar polymers, or blends thereof. In some aspects, the first material can comprise a substantially rigid or non-deformable material, such as a substantially rigid plastic material.

An interior surface **111** of the tube wall **107** proximate the first end **103** can be overmolded with a second material, which may be chosen from flexible elastomeric materials, to form the flexible bottom **109**. Examples of suitable flexible or deformable elastomeric materials include, but are not limited to thermoplastic and thermoset elastomers, such as silicon-based polymers (e.g., polydimethylsiloxane) or multi-block elastomer alloys (e.g., Versaflex^{™} products from PolyOne, such as CL2250 or HC MT222), to name a few. According to various aspects, the first material can have a higher rigidity than that of the second material, e.g., the tubular body **107** can be substantially rigid while the bottom **109** can be substantially flexible and/or deformable.

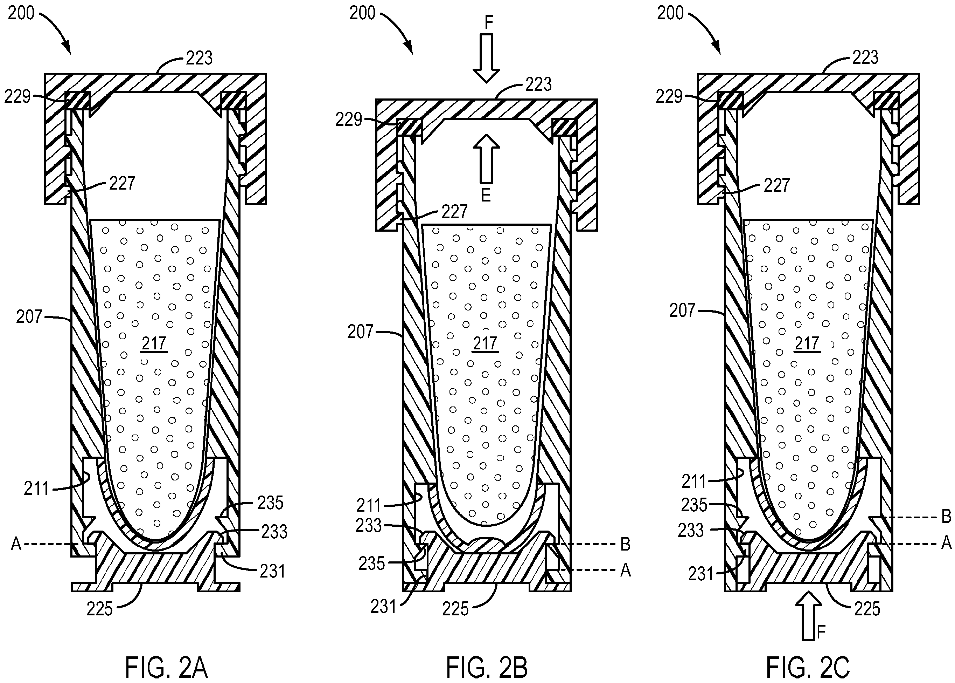

As used herein the term "end" is intended to refer to an edge or boundary of tubular body which may be open or closed. For instance, the second end can be open or closed by a cap. Similarly, the first end can be closed by a flexible bottom or other feature. The flexible bottom can be recessed inside the tubular body or can protrude past the first end. Referring to FIGS. 1A-B, the tubular body can be "closed" by the overmolded flexible bottom recessed inside the tubular body proximate the first end, while the first end can remain "open" such that the flexible bottom can be contacted by the user to eject the frozen sample. Referring to FIGS. 2A-C, the tubular body can be "closed" by the overmolded flexible bottom recessed inside the tubular body proximate the first end, and the first end can also be "closed" by the integral ejector feature.

The term "overmolded" is intended to denote that the second material is applied and bonded to the first material, e.g., by melt processing and subsequent bonding, or vice versa. The bond between the first and materials can be a mechanical, melt, and/or chemical bond, according to various aspects. Mechanical bonding can entail, for instance, softening or melting the second material and flowing the material onto a substrate (e.g., a tubular body) comprising the first material, wherein the second material flows into holes, undercuts, channels, or other features on the substrate surface. After solidifying, the overmolded second material can thus be mechanically connected or interlocked with the substrate or tubular body. In certain instances, the overmolded material can form a mechanical lock, such as an interlocking tongue and groove, which can have a relatively high strength. Additionally, the use of mechanical bonding can provide a wider range of choices with respect to the first and second materials (e.g., in terms of melting temperatures, chemical composition, etc.).

Melt bonding can be derived from adhesion between the surfaces of two materials with relatively similar melting points. For example, at a given overmolding temperature, both materials may at least partially melt and mutually solvate at their interface to form a bond. Some degree of chemical similarity may enhance a melt bond thus formed, although it is not necessary. Chemical bonding can occur, for example, in the case of two chemically compatible materials, by a chemical reaction at the interface to bond the materials together. Chemical and melt bonding can provide a relatively strong bond between the two materials as compared to mechanical bonding but may also, in some instances, narrow the range of choices for the first and second materials.

Overmolding can be achieved, for example, by a two-shot molding process. In such a process, a first injection molding machine can be used to mold a substrate (e.g., tubular body) from the first material. The substrate can then be transferred to a second injection molding machine in which the second material is molded onto the substrate. In various aspects, the substrate may have an elevated temperature above room temperature (from the first injection molding step), and may be in a semi-solid or gel phase. Thus, melt and chemical bonding may be improved between the first and second materials using such a two-shot molding process. Of course, the substrate can also be provided with features to provide one or more mechanical bonds or locks.

According to various aspects, the flexible bottom **109** can be overmolded onto an interior surface **111** of the tube wall **107**. In some aspects, the second material can be molded directly on to the interior surface. In other aspects, the second material can be molded onto a feature **113** of the interior surface (as shown in FIGS. 1A-B). As such, as used herein, the term "interior surface" of the tube wall is intended to denote an interior surface of the tube wall, a feature on the interior surface, a surface of the feature, or any combination thereof.

The feature **113** can, in various aspects, be constructed of the same material as the tube wall. The feature **113** can be a protrusion or recess having any desired shape or size. For example, the feature **113** can have a wedge-shaped cross-section (as depicted) such that a portion of the feature proximate the first end **103** can extend or jut into the cavity **101**. Of course any other feature shape and/or size is possible and envisioned as falling within the scope of the disclosure. Any surface of the feature **113** can be used as a bonding interface between the first material and the second material. For instance, as illustrated in FIGS. 1A-B, the feature can comprise a surface **119a** forming an angle (e.g. a perpendicular angle) with to the tube wall **107** and this surface can comprise the bonding interface **121**. It should be noted that surface **119b**, also forming an angle with the tube wall **107**, could also be used as a bonding interface, or any other portion of the interior surface **111** of the tube wall **107**, without limitation.

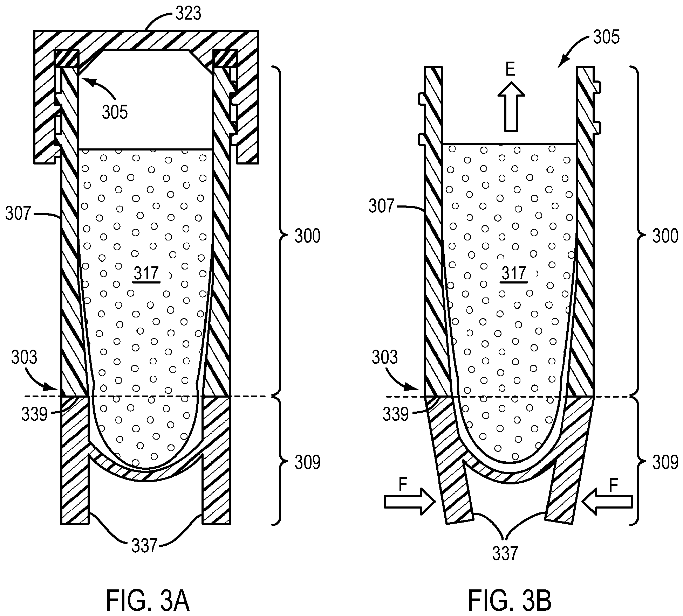

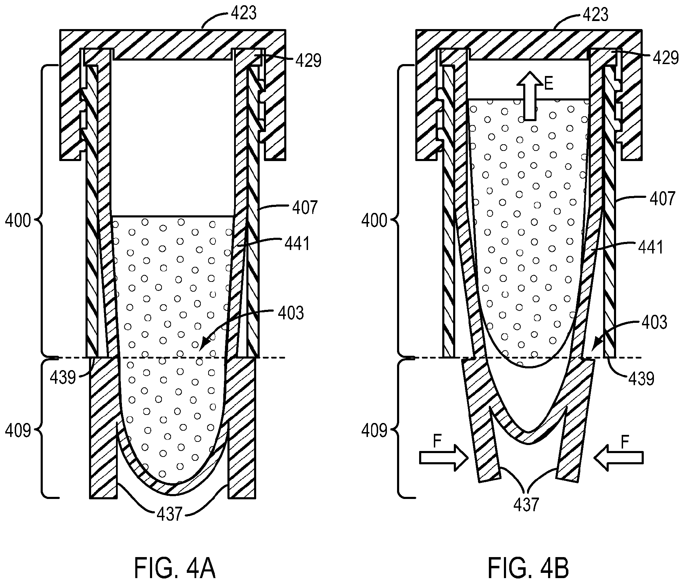

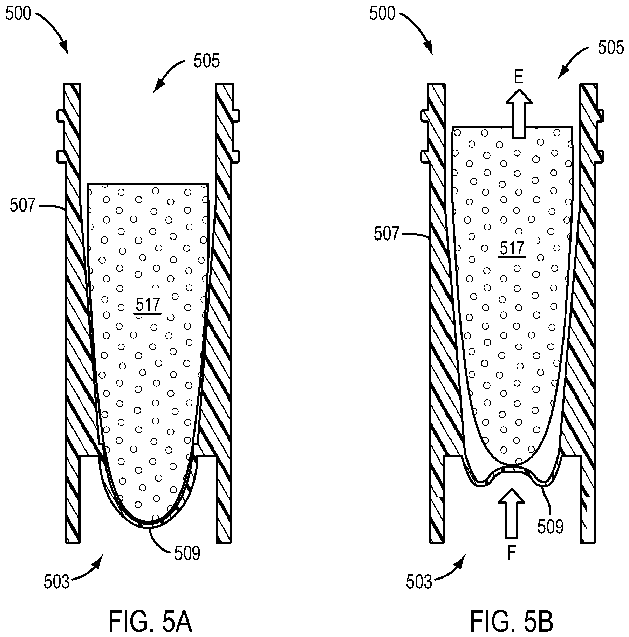

As depicted in **FIG. 1B**, a force **F** can be applied to the flexible bottom **109** to dislodge or eject a sample **117** from the vial assembly. In certain aspects not forming part of the present invention, a component external to the vial assembly can be brought into contact with the flexible bottom to apply the force **F**. For example, a user can depress the flexible bottom with a thumb or finger, or with a separate device, such as a plunger, ejector, or any other suitable object. In other aspects, an ejector may be integral to the vial assembly, for instance, as depicted in **FIGS. 2-4** and **6**. The ejector can be a separate piece fitted to the vial assembly (e.g., FIGS. 2A-C) or can be a part of the tubular body, e.g., the tube wall (e.g., FIGS. 5A-B). In other aspects, the ejector can be part of or otherwise attached to the flexible bottom (e.g., FIGS 3A-B and **4A-B**).

FIGS. 2A-C depict cross-sectional views of a vial assembly of FIGS 1A-B further equipped with a cap **223** and an integral ejector **225.** In some aspects, the cap **223** can comprise threading **227** on an internal or external surface, configured to mate with threading **215** on the tube wall **207**. A seal **229** may also be present to enhance and/or tighten the closure between the tubular body and the cap. The cap **223** and/or ejector **225** can be constructed from any suitable material, for example, materials similar to or different from the material from which the tube walls **207** are constructed. In non-limiting aspects, the cap and/or ejector can comprise a first material chosen from polyolefins (e.g., polyethylene or polypropylene), fluoropolymers (e.g., polytetrafluoroethylene), polycarbonates, polyesters, polystyrenes, and other similar polymers, or blends thereof. Of course the additional depicted features in FIGS. 2A-C are not limited to operation with the vial of FIGS. 1A-B and any features disclosed herein can be interchanged with any other vial assembly.

Vial assemblies may be equipped with bar codes for tracking samples, for example, a bar code may be placed on the bottom of a cryotube. Bar codes can be printed, for example, on a bar code disk that can be located at the base of the tube. In some aspects, a bar code disk can be used as an integral ejector **225**. Of course, an integral ejector having a different shape and/or size can be used, with or without the presence of a barcode, these features being interchangeable without limitation. The integral ejector **225** can be retained in a first position **A**, in which the ejector does not apply an ejecting force on the flexible bottom. The interior surface **211** of the tube wall **207** can, for example, be equipped with one or more features **231** for retaining the ejector **225**. Such features can comprise protrusions or recesses on or in the tube wall **207**. As illustrated, protrusions jut out from the interior surface **211**; however, a different configuration in which the inner surface is contoured to create recesses is also envisioned, as well as any other feature capable of retaining the ejector **225**. The integral ejector **225** can comprise one or more corresponding features **233** configured to hold or retain the ejector in the desired position (e.g., position **A** as depicted in **FIG. 2A**)**.**

As shown in **FIG. 2B**, a force **F** can be applied to the top of the vial assembly such that the integral ejector **225** is dislodged from the first position and forced into a second position **B**, in which the ejector contacts the flexible bottom thereby applying an ejecting force to the sample **217** in direction **E**, e.g., dispensing the sample via second end **205**. For example, a user can press down on the vial cap **223**, or up on the ejector **225** to generate an ejecting force. In some aspects, one or more second features **235** can be included on the interior surface **211** to retain the ejector **225** in position **B**. As depicted in **FIG. 2C**, the integral ejector **225** can be configured to fully nest within the tube wall **207**, in which case force **F** can be applied to the ejector, e.g., by depressing or pushing the ejector inward. Alternatively, as depicted in FIGS. 2A-B, the ejector **225** can be configured to extend at least partially beyond the tube wall **207**, in either or both positions **A** or **B**, in which case force **F** can be applied to the top or bottom of the vial assembly to eject the sample.

Additional exemplary vial assemblies are depicted in FIGS. 3A-B. In the illustrated embodiments, the vial assembly comprises a tubular body **300** comprising a first end **303**, a second end **305** (labeled in **FIG. 3B**, closed by cap **323** in **FIG. 3A**), and a tube wall **307**. A flexible bottom **309** is overmolded on the first end **303** (boundary indicated by the dashed line), e.g., extending across the tube wall and closing the first end of the tubular body **300**. The flexible bottom **309** is equipped with two or more flanges **337**, which can be configured to serve multiple functions, e.g., providing a stand for the vial and/or a means for storing the vial within a rack or stand. As shown in **FIG. 3B**, the flanges **337** are configured to bend inwardly upon application of force F to apply pressure to the flexible bottom **309**. For instance, the flanges **337** may be used as a hinge to pinch or otherwise impinge upon the flexible bottom **309** such that the frozen sample **317** is dislodge or ejected from the vial in direction **E**, e.g., dispensing the sample from the second end **305**.

As discussed above, the tubular body comprises a first material and the flexible bottom can comprise a second elastomeric material overmolded on the first opening of the tubular body. In some embodiments, a bottom outer surface **339** (see **FIG. 3B**) of the tube wall **307** can serve as the bonding interface (not labeled), although the flexible bottom can also be overmolded on other surfaces, such as the interior or exterior surfaces of the tube wall **307**. The flexible bottom **309** can, for instance, span the entire periphery of the first end **303** of the tubular body. In some embodiments, as illustrated, the flexible bottom **309** and flanges **337** can form a unitary overmolded piece. The flanges **337** can extend, e.g., from the bottom surface **339** of the tube wall **307** and can, in some instances, form a full or partial extension of the tube wall **307**. For example, each flange can be semi-circular or arcuate in shape, e.g., approximating the peripheral contour of the tube wall **307**, and can provide a stand for the vial assembly such that the flexible bottom **309** is not impinged upon by a surface on which the vial rests when in the upright position.

In further embodiments, as illustrated in FIGS. 4A-B, the vial assembly can comprise a tubular body **400** comprising a first end **403**, a second end **305** (not labeled, closed by cap **423**), and a tube wall **407**. A flexible liner **441** can be inserted into or otherwise positioned in the cavity (not labeled) of the tubular body. In some non-limiting embodiments, the liner **441** can be overmolded onto an interior and/or exterior surface of the tube wall **407**. The flexible liner **441** can extend past the first end **403** (boundary indicated by the dashed line) of the tubular body **400**, e.g., providing a flexible bottom **409** closing the first end of the tubular body **400**.

As in FIGS. 3A-B, the flexible bottom **409** can be equipped with two or more flanges **437**, which can be configured to serve multiple functions, e.g., providing a stand for the vial and/or a means for storing the vial within a rack or stand. As shown in **FIG. 4B**, the flanges **437** are configured to bend inwardly upon application of force **F** to apply pressure to the flexible bottom **409**. For instance, the flanges **437** may be used as a hinge to pinch or otherwise impinge upon the flexible bottom **409** such that the frozen sample **417** is dislodge or ejected from the vial in direction **E**. Furthermore, as illustrated in **FIG. 4B**, the cap need not be removed to exert force on the flexible bottom to at least partially dislodge the frozen sample or pellet. As such, for any vial assemblies disclosed herein, the cap can be removed followed by applying force to the flexible bottom or vice versa.

The tubular body comprises a first material and the flexible liner comprises a second elastomeric material inserted into the cavity of the tubular body. In some embodiments, the flexible liner **441** can also serve as a seal **429** between a top surface (not labeled) of the tubular body and the cap **423**. For instance, the second end (not labeled) can comprise a lip which can engage the flexible liner **441**, e.g., such that the liner sits atop, rests on, or abuts the lip. In the closed position, e.g., when the cap **423** is engaged to the tubular body via threading or another closing feature, at least a portion of the flexible liner **441** can rest between the cap **423** and tubular body **400** to enhance the integrity of the vial assembly. Suitable elastomeric materials can include, for example, thermoplastic and thermoset elastomers, such as silicon-based polymers (e.g., polydimethylsiloxane) or multi-block elastomer alloys (e.g., Versaflex^{™} products from PolyOne, such as CL2250 or HC MT222), to name a few.

In some embodiments, as illustrated, the flexible liner **441**, bottom **409**, and flanges **437** can form a unitary piece. The flanges **437** can extend, e.g., from the bottom surface **439** of the tube wall **407** and can, in some instances, form a full or partial extension of the tube wall **407**. For example, each flange can be semi-circular or arcuate in shape, e.g., approximating the peripheral contour of the tube wall **407**, and can provide a stand for the vial assembly such that the flexible bottom **409** is not impinged upon by a surface on which the vial rests when in the upright position.

In alternative aspects not forming part of the present invention, as illustrated in FIGS. 5A-B, a vial assembly can comprise a unitary piece constructed from a single (e.g., first) material. Similar to the other vial assemblies, a tubular body **500** can comprise a first end **503,** a second end **505**, a tube wall **507**, and a flexible bottom **509**. As depicted in **FIG. 5B**, a force **F** can be applied to the flexible bottom **509** to dislodge or eject a sample **517** from the vial assembly. In certain aspects, a component external to the vial assembly can be brought into contact with the flexible bottom to apply the force **F**. For example, a user can depress the flexible bottom with a thumb or finger, or with a separate device, such as a plunger, ejector, or any other suitable object. In other aspects, an ejector may be integral to the vial assembly.

In non-limiting aspects, the tubular body **500**, e.g., flexible bottom **509** and tube wall **507**, can comprise a first material chosen from polyolefins (e.g., polyethylene or polypropylene), fluoropolymers (e.g., polytetrafluoroethylene), polycarbonates, polyesters, polystyrenes, and other similar polymers, or blends thereof. In certain aspects, the tubular body **500** can comprise a material which, at a sufficiently high thickness can provide rigidity (e.g., for the tube wall **507**), but at a sufficiently low thickness can provide flexibility (e.g., for the bottom **509**). For instance, various portions of the tubular body **500** can have a reduced wall thickness to enhance malleability of the tube in those locations, e.g., at the bottom. Whereas the tube wall **507** can have a thicker sidewall, the bottom **509** may have a reduced wall thickness, which may facilitate bending, flexing, and/or pinching of the tube in this location to encourage dislodgment of the frozen sample from the tube.

According to various aspects, the thickness of the tube wall **507** can range from about 0.05 cm to about 0.2 cm, such as from about 0.06 cm to about 0.15 cm, or from about 0.075 cm to about 0.125 cm, including all ranges and subranges therebetween, whereas the thickness of the bottom **509** can range from about 0.025 cm to about 0.1 cm, such as from about 0.03 cm to about 0.075 cm, from about 0.04 cm to about 0.07 cm, or from about 0.05 cm to about 0.06 cm, including all ranges and subranges therebetween. In certain aspects, a ratio of the thickness of the tube wall to the thickness of the bottom can range from about 2:1 to about 20:1, such as from about 3:1 to about 15:1, from about 4:1 to about 12:1, from about 5:1 to about 10:1, or from about 6:1 to about 8:1, including all ranges and subranges therebetween.

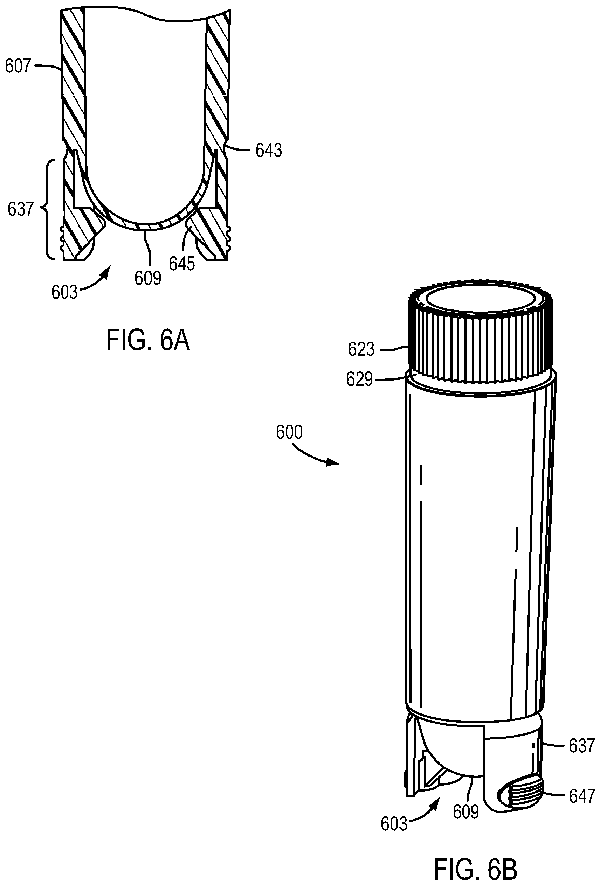

Whether the tubular body comprises one material (e.g., as illustrated in FIGS. 5A-B) or more than one material (e.g., as illustrated in FIGS. 1A-B), the first end of the tubular body can be equipped with one or more features for promoting disengagement of the frozen sample from the vial assembly. The first end of the tubular body **600** and will be discussed in more detail with reference to the cross-sectional image depicted in **FIG. 6A**. The vial assembly is provided with flanges **637** proximate the first end **603**. As illustrated in **FIG. 6A**, the bottom **609** can be substantially rounded, although other shapes are possible and envisioned as falling within the scope of the disclosure. The flanges **637** can be part of the tubular body (as illustrated), e.g., constructed from the same material, or separate from the tubular body, e.g., constructed from a different material. According to the invention, the flanges **637** are configured to bend inwardly upon application of force to apply pressure to the bottom **609**. For instance, the flanges **637** may be used as a hinge to pinch or otherwise impinge on the bottom **609**.

Further features may be added to the flanges **637** to enhance the ability to dislodge the frozen sample from the vial. In some embodiments, the tube wall **607** of the vial can be thinned in a region **643** proximate the bottom **609**. Thinning of the tube wall **607** in this location may enhance the ability of the flanges **637** to hinge inwardly. Additionally, the flanges **637** can be equipped with gussets or inward protrusions **645** that can increase the force applied to the bottom **609**. Lateral or perpendicular force applied to the flanges **637** can be redirected by the gussets **645** to the bottom, e.g., providing a vertical or parallel force pushing into the bottom **609** to dislodge the pellet.

**FIG. 6B** illustrates a perspective view of the exterior of a vial assembly according to various aspects of the disclosure. In the closed position, the cap **623** can be engaged with the tubular body **600** via seal **629**. The first end **603** of the tubular body **600** is optionally equipped with flanges **637** for standing the vial upright and/or for disengaging the frozen sample from the vial. The flanges **637** can, in some embodiments, include a textured region **647**, which can be raised for enhanced gripping by a user during use (e.g., finger grips) and/or for insertion into a storage rack or block, or recessed for fitting into standard tube racks.

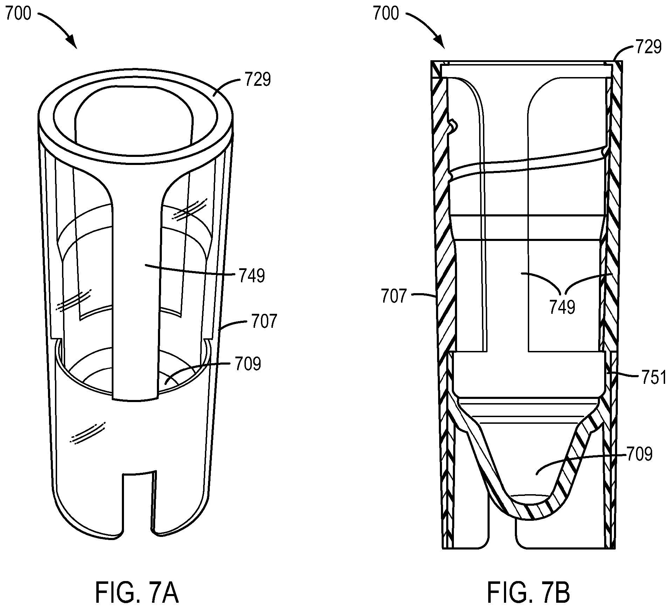

Gripping features can, in some embodiments, advantageously enable a user to hold onto the vial assembly during handling and/or opening. For instance, slippery condensate on the frozen vial and/or a loss of dexterity due to personal protection equipment worn by the user can make handling and/or opening the vial assembly difficult. According to various embodiments, it may be advantageous to provide gripping or textured features on the exterior of the tubular body as shown in FIGS. 7A-B.

Furthermore, as discussed above, a seal is often incorporated to improve closure of the vial between the tubular body and the cap. Currently, a secondary process is employed to add a seal or gasket to the vial assembly. However, such secondary seals tend to become brittle when exposed to cryogenic conditions, such as liquid nitrogen. Moreover, the additional processing steps can complicate the process and/or increase the expense of the final product. It would thus additionally be advantageous to provide sealing features during an overmolding step, e.g., during formation of a flexible bottom and/or gripping features on the vial assembly, using materials with enhanced ductility and/or reduced brittleness.

Such gripping and sealing features will be discussed with reference to FIGS. 7A-B, which depict perspective and cross-sectional views of a vial assembly according to various non-limiting embodiments. According to some embodiments, such as the vial assemblies depicted in **FIGS. 1-3** including overmolded flexible bottoms, the overmolding process can also be used to supply gripping and/or sealing features on the exterior of the tubular body. For example, as illustrated in **FIGS. 7A-****B,** during an overmolding process used to bond the flexible bottom **709** to the tube wall **707,** the second material can also be overmolded on one or more portions of the tube wall exterior, such as gripping features **749** and/or sealing features **729.**

The sealing and/or gripping features **729, 749** can be bonded to the tubular body **700** via mechanical, chemical, and/or melt bonding. In some embodiments, the gripping features **749** can be at least partially mechanically bonded to the tube wall **707** via one or more channels, grooves, or troughs (not illustrated) into which the second material can flow after being melted or softened. For instance, during an overmolding process, the flexible bottom can be formed at the first end and the second material can then flow along one or more troughs or channels created on a surface of the tubular body. According to various embodiments, after forming the flexible bottom at the first end, a band **751** can be formed along an internal or external periphery of the tube wall, e.g., by flowing the second material into a trough formed on a surface of the tube wall (interior surface illustrated). One or more gripping features **749,** which may be optionally textured, can then be formed by flowing the second material into one or more grooves or channels running along the length or a portion of the length of the exterior surface of the tube wall. Finally, a sealing feature or gasket **729** can be formed by flowing the second material over the outer periphery or lip of the second end. In this non-limiting embodiment, the surface area of attachment and the various mechanical interlocking joints formed by the channels, troughs, and/or gasket can create a very strong bond between the first and second materials.

While FIGS. 7A-B illustrate three gripping features **749** substantially spaced apart and having substantially the same shape, it is to be understood that any shape and/or number of features can be provided using different channels, grooves, and/or troughs. In some embodiments, one or two gripping features can be included, or more than three gripping features can be included, such as four, five, six, or more features. Additionally, while gripping features **749** are illustrated as extending along the entire length of the tubular body, it is to be understood that such features can have any desired length. Moreover, while FIGS. 7A-B illustrate such features in combination with a vial assembly comprising an overmolded flexible bottom, these features can also be combined with other vial assemblies disclosed herein, such as the embodiments and aspects illustrated in FIGS. 4A-B or **5A-B.**

The vial assemblies, including the tubular bodies, caps, liners, bottoms, grips, etc., can be manufactured from any materials suitable for cryopreservation applications, for instance, the first and second materials disclosed herein. In some embodiments, the first and/or second material can be a thermally conductive material, e.g., a material capable of transferring heat from a user's hands or another heat source to the frozen sample to further facilitate removal of the sample from the vial assembly. According to various embodiments, the tubular body and cap of the vial assembly can comprise the same or different materials. In additional aspects, the tube wall and flexible bottom of the tubular body can comprise the same or different materials. The cap may comprise a substantially rigid material, whereas the tube wall can comprise a similarly rigid or relatively less rigid material. Likewise, the flexible bottom can comprise a material having less rigidity as compared to the tube wall and/or cap.

Additional optional features can be included in the vial assembly disclosed herein, e.g. for improved ease of handling, heat transfer, and/or sealing of the vial. Some exemplary non-limiting features are described, for example, the caps disclosed in U.S. Provisional Application No. 62/255,633 entitled "CRYOGENIC VIAL ASSEMBLIES" filed on November 16, 2015.

### Methods

Methods disclosed herein, but not forming part of the claimed invention, can include methods for preparing a frozen sample in a vial assembly and methods for removing a frozen sample from a vial assembly. Methods for preparing and/or storing frozen samples can include introducing a liquid sample into a cavity of a tubular body; engaging an open end of the tubular body with a cap to form a sealed vial assembly; and freezing the sealed vial assembly. Methods for removing frozen samples from a vial assembly can include applying force to the flexible bottom of the vial assembly to loosen and/or dislodge the frozen sample from the tube. Force can be applied, for example, using an external ejector such as a thumb or finger of the user or any other component, such as a plunger or other similar tool. Force can also be applied using an integral ejector, e.g., part of the vial assembly, such as an ejector retained by one or more features of the vial assembly and/or flanges making up one or more parts of the assembly. Of course, it is to be understood that the features disclosed herein with respect to the vial assembly are intended to similarly apply to the methods disclosed herein, such that a method for preparing or removing the sample can employ or utilize one or more features described with respect to the vial assembly.

According to various aspects, a frozen sample, e.g. comprising a biological sample such as cells or tissues, can be introduced into a vial assembly disclosed herein. For instance, a predetermined amount of a liquid sample can be poured the second end of the tubular body. The liquid sample can be added, in various aspects, in any desired amount. By way of non-limiting example, the liquid sample can at least partially fill the cavity of the tubular body, e.g., at least about 10%, 20%, 30%, 40%, 50%, 60%, 70%, 80%, 90%, or even 100% of the cavity may be filled with the liquid sample.

A cap can then be coupled to the second end of the tubular body, e.g., by rotating the cap such that threading on the cap engages at least a portion of threading on the tubular body, by snap-fit, or by a like mechanism. Coupling of the cap to the tubular body, e.g., by rotation, can be carried out until the cap is snugly fit to the tubular body, for instance, until a bottom surface of the cap abuts a top surface or lip of the tubular body or until the cap abuts a seal disposed between the cap and the lip of the tubular body. Upon sealing the vial assembly in the closed position, the vial assembly and sample contained therein can be frozen, e.g., at a temperature below the freezing point of the sample. The vial assembly can, for instance, be frozen in an upright position such that the liquid is in contact with a bottom internal surface of the cavity. The vial assembly can also be frozen in any other position, such as a horizontal or inverted position, such that the liquid is in contact with a top internal surface and/or the bottom internal surface of the cavity. Regardless of orientation, the sample can be frozen such that application of force to the flexible bottom at least partially dislodges the sample from the vial assembly.

Removal of the frozen sample from the vial assembly can be achieved by disengaging the cap from the tubular body either before or after applying force to the flexible bottom to dislodge the frozen sample from the interior surfaces of the vial assembly. According to various aspects, the flexible bottom can be compressed by squeezing two or more flanges proximate the closed end. The flanges can apply lateral and/or vertical (e.g., inward and/or upward) force to the bottom of the tubular body to push the frozen sample out of the vial. An integral or external ejector can also be pressed against the flexible bottom to push the frozen sample out of the vial, either by pushing the ejector into the flexible bottom or otherwise pressing the flexible into the ejector. The application of vertical and/or lateral force can serve to loosen the frozen sample from the internal surfaces of the tubular body such that the sample can be more easily ejected from the vial assembly.

It will be appreciated that the various disclosed examples may involve particular features, elements or steps that are described in connection with that particular example. It will also be appreciated that a particular feature, element or step, although described in relation to one particular example, may be interchanged or combined with alternate examples in various non-illustrated combinations or permutations.

## Claims

1. A vial assembly comprising:
a tubular body (100;300;400;500;600;700) comprising a cavity (101), a first end (103;303;403;503;603), a second end (105;205;305;505) for dispensing a sample (117;217;317;417;517), and a tube wall (107;207;307;407;507;607;707) comprising a first material; and
a flexible bottom (109;309;409;509;609;709) comprising a second elastomeric material overmolded on an interior surface (111;211) of the tube wall (107;207;307;407;507;607;707) proximate the first end (103;303;403;503;603),
wherein the flexible bottom (109;309;409;509;609;709) does not extend past the first end (103;303;403;503;603) of the tubular body (100;300;400;500;600;700),
**characterised in that** the tube wall (107;207;307;407;507;607;707) comprises at least two flanges (337;437;637) proximate the flexible bottom (109;309;409;509;609;709), wherein the flanges (337;437;637) are configured to hinge inwardly upon application of force to apply pressure to the flexible bottom (109;309;409;509;609;709).

2. The vial assembly of claim 1, wherein the flanges (337;437;637) further comprise at least one gusset (645) configured to apply force to the flexible bottom (109;309;409;509;609;709).

3. A vial assembly comprising:
a tubular body (100;300;400;500;600;700) comprising a cavity (101), a first end (103;303;403;503;603), a second end (105;205;305;505), and a tube wall (107;207;307;407;507;607;707) comprising a first material; and
a flexible bottom (109;309;409;509;609;709) comprising a second elastomeric material overmolded on the first end (103;303;403;503;603) of the tubular body (100;300;400;500;600;700),
**characterised in that** the flexible bottom (109;309;409;509;609;709) further comprises at least two flanges (337;437;637) attached to the tube wall (107;207;307;407;507;607;707) and configured to hinge inwardly upon application of force to apply pressure to a surface of the flexible bottom (109;309;409;509;609;709).

4. A vial assembly comprising:
a substantially rigid tubular body (100;300;400;500;600;700) comprising a cavity, a first end (103;303;403;503;603), a second end (105;205;305;505), and a tube wall (107;207;307;407;507;607;707) comprising a first material; and
a flexible liner (441) comprising a second elastomeric material positioned in the cavity of the tubular body (100;300;400;500;600;700) and extending past the first end (103;303;403;503;603) of the substantially rigid tubular body (100;300;400;500;600;700),
**characterised in that** the flexible liner (441) further comprises at least two flanges (337;437;637), and wherein the flanges (337;437;637) are configured to hinge inwardly upon application of force to apply pressure to a surface of the flexible liner (441).

5. The vial assembly of claim 4, wherein the second end (105;205;305;505) comprises a lip and wherein the flexible liner (441) covers at least a portion of the lip.

6. The vial assembly of claim 4 or 5, further comprising a cap (223;323;423;623), wherein the flexible liner (441) is configured to form a seal (229;429;629;729) between the lip and the cap (223;323;423;623) in a closed position.

7. The vial assembly of any one of claims 4-6, wherein the flexible liner (441) is overmolded on an interior or exterior surface of the tube wall (107;207;307;407;507;607;707).

## Patentansprüche

1. Glasfläschchenanordnung, umfassend:
einen rohrförmigen Körper (100;300;400;500;600;700), der einen Hohlraum (101), ein erstes Ende (103;303;403;503;603), ein zweites Ende (105; 205; 305; 505) zum Abgeben einer Probe (117;217;317;417;517) und eine ein erstes Material umfassende Rohrwand (107;207;307;407;507;607;707) umfasst; und
einen flexiblen Boden (109;309;409;509;609;709), der ein zweites Elastomermaterial umfasst, das auf eine Innenfläche (111;211) der Rohrwand (107;207;307;407;507;607;707) in der Nähe des ersten Endes (103;303;403;503;603) aufgeformt ist,
wobei sich der flexible Boden (109;309;409;509;609;709) nicht über das erste Ende (103;303;403;503;603) des rohrförmigen Körpers (100;300;400;500;600;700) hinaus erstreckt,
**dadurch gekennzeichnet, dass** die Rohrwand (107;207;307;407;507;607;707) mindestens zwei Flansche (337;437;637) in der Nähe des flexiblen Bodens (109;309;409;509;609;709) umfasst, wobei die Flansche (337;437;637) so konfiguriert sind, dass sie bei Krafteinwirkung nach innen klappen, um Druck auf den flexiblen Boden (109;309;409;509;609;709) auszuüben.

2. Glasfläschchenanordnung nach Anspruch 1, wobei die Flansche (337;437;637) ferner mindestens einen Keil (645) umfassen, der so konfiguriert ist, dass er Kraft auf den flexiblen Boden (109;309;409;509;609;709) ausübt.

3. Glasfläschchenanordnung, umfassend:
einen rohrförmigen Körper (100;300;400;500;600;700), der einen Hohlraum (101), ein erstes Ende (103;303;403;503;603), ein zweites Ende (105;205;305;505) und eine ein erstes Material umfassende Rohrwand (107;207;307;407;507;607;707) umfasst; und
einen flexiblen Boden (109;309;409;509;609;709), der ein zweites Elastomermaterial umfasst, das auf das erste Ende (103;303;403;503;603) des rohrförmigen Körpers (100;300;400;500;600;700) aufgeformt ist,
**dadurch gekennzeichnet, dass** der flexible Boden (109;309;409;509;609;709) ferner mindestens zwei Flansche (337;437;637) umfasst, die an der Rohrwand (107;207;307;407;507;607;707) befestigt sind und so konfiguriert sind, dass sie bei Krafteinwirkung nach innen klappen, um Druck auf eine Oberfläche des flexiblen Bodens (109;309;409;509;609;709) auszuüben.

4. Glasfläschchenanordnung, umfassend:
einen im Wesentlichen starren rohrförmigen Körper (100;300;400;500;600;700), der einen Hohlraum, ein erstes Ende (103;303;403;503;603), ein zweites Ende (105;205;305;505) und eine ein erstes Material umfassende Rohrwand (107;207;307;407;507;607;707) umfasst; und
eine flexible Auskleidung (441), die ein zweites Elastomermaterial umfasst, das im Hohlraum des rohrförmigen Körpers (100;300;400;500;600;700) positioniert ist und sich über das erste Ende (103;303;403;503;603) des im Wesentlichen starren Rohrkörpers (100;300;400;500;600;700) hinaus erstreckt,
**dadurch gekennzeichnet, dass** die flexible Auskleidung (441) ferner mindestens zwei Flansche (337;437;637) umfasst und wobei die Flansche (337;437;637) so konfiguriert sind, dass sie bei Krafteinwirkung nach innen klappen, um Druck auf eine Oberfläche der flexiblen Auskleidung (441) auszuüben.

5. Glasfläschchenanordnung nach Anspruch 4, wobei das zweite Ende (105;205;305;505) eine Lippe umfasst und wobei die flexible Auskleidung (441) mindestens einen Teil der Lippe bedeckt.

6. Glasfläschchenanordnung nach Anspruch 4 oder 5, ferner umfassend eine Kappe (223;323;423;623), wobei die flexible Auskleidung (441) so konfiguriert ist, dass sie eine Dichtung (229;429;629;729) zwischen der Lippe und der Kappe (223;323;423;623) in geschlossener Position bildet.

7. Glasfläschchenanordnung nach einem der Ansprüche 4-6, wobei die flexible Auskleidung (441) auf eine Innen- oder Außenfläche der Rohrwand (107;207;307;407;507;607;707) aufgeformt ist.

## Revendications

1. Ensemble de fiole, comprenant :
un corps tubulaire (100 ; 300 ; 400 ; 500 ; 600 ; 700) comprenant une cavité (101), une première extrémité (103 ; 303 ; 403 ; 503 ; 603), une seconde extrémité (105 ; 205 ; 305 ; 505) pour distribuer un échantillon (117 ; 217 ; 317 ; 417 ; 517), et une paroi de tube (107 ; 207 ; 307 ; 407 ; 507 ; 607 ; 707) comprenant un premier matériau ; et
un fond souple (109 ; 309 ; 409 ; 509 ; 609 ; 709) comprenant un second matériau élastomère surmoulé sur une surface intérieure (111 ; 211) de la paroi de tube (107 ; 207 ; 307 ; 407 ; 507 ; 607 ; 707) à proximité de la première extrémité (103 ; 303 ; 403 ; 503 ; 603),
dans lequel le fond souple (109 ; 309 ; 409 ; 509 ; 609 ; 709) ne s'étend pas au-delà de la première extrémité (103 ; 303 ; 403 ; 503 ; 603) du corps tubulaire (100 ; 300 ; 400 ; 500 ; 600 ; 700),
**caractérisé en ce que** la paroi de tube (107 ; 207 ; 307 ; 407 ; 507 ; 607 ; 707) comprend au moins deux rebords (337 ; 437 ; 637) à proximité du fond souple (109 ; 309 ; 409 ; 509 ; 609 ; 709), dans lequel les rebords (337 ; 437 ; 637) sont configurés pour s'articuler vers l'intérieur lors de l'application d'une force pour appliquer une pression sur le fond souple (109 ; 309 ; 409 ; 509 ; 609 ; 709).

2. Ensemble de fiole selon la revendication 1, dans lequel les rebords (337 ; 437 ; 637) comprennent en outre au moins un gousset (645) configuré pour appliquer une force au fond souple (109 ; 309 ; 409 ; 509 ; 609 ; 709).

3. Ensemble de fiole, comprenant :
un corps tubulaire (100 ; 300 ; 400 ; 500 ; 600 ; 700) comprenant une cavité (101), une première extrémité (103 ; 303 ; 403 ; 503 ; 603), une seconde extrémité (105 ; 205 ; 305 ; 505) , et une paroi de tube (107 ; 207 ; 307 ; 407 ; 507 ; 607 ; 707) comprenant un premier matériau ; et
un fond souple (109 ; 309 ; 409 ; 509 ; 609 ; 709) comprenant un second matériau élastomère surmoulé sur la première extrémité (103 ; 303 ; 403 ; 503 ; 603) du corps tubulaire (100 ; 300 ; 400 ; 500 ; 600 ; 700),
**caractérisé en ce que** le fond souple (109 ; 309 ; 409 ; 509 ; 609 ; 709) comprend en outre au moins deux rebords (337 ; 437 ; 637) fixés à la paroi de tube (107 ; 207 ; 307 ; 407 ; 507 ; 607 ; 707) et configurés pour s'articuler vers l'intérieur lors de l'application d'une force pour appliquer une pression sur une surface du fond souple (109 ; 309 ; 409 ; 509 ; 609 ; 709).

4. Ensemble de fiole, comprenant :
un corps tubulaire sensiblement rigide (100 ; 300 ; 400 ; 500 ; 600 ; 700) comprenant une cavité, une première extrémité (103 ; 303 ; 403 ; 503 ; 603), une seconde extrémité (105 ; 205 ; 305 ; 505), et une paroi de tube (107 ; 207 ; 307 ; 407 ; 507 ; 607 ; 707) comprenant un premier matériau ; et
un chemisage souple (441) comprenant un second matériau élastomère positionné dans la cavité du corps tubulaire (100 ; 300 ; 400 ; 500 ; 600 ; 700) et s'étendant au-delà de la première extrémité (103 ; 303 ; 403 ; 503 ; 603) du corps tubulaire sensiblement rigide (100 ; 300 ; 400 ; 500 ; 600 ; 700),
**caractérisé en ce que** le chemisage souple (441) comprend en outre au moins deux rebords (337 ; 437 ; 637), et dans lequel les rebords (337 ; 437 ; 637) sont configurés pour s'articuler vers l'intérieur lors de l'application d'une force pour appliquer une pression sur une surface du chemisage souple (441).

5. Ensemble de fiole selon la revendication 4, dans lequel la seconde extrémité (105 ; 205 ; 305 ; 505) comprend une lèvre et dans lequel le chemisage souple (441) recouvre au moins une partie de la lèvre.

6. Ensemble de fiole selon la revendication 4 ou 5, comprenant en outre un capuchon (223 ; 323 ; 423 ; 623), dans lequel le chemisage souple (441) est configuré pour former un joint (229 ; 429 ; 629 ; 729) entre la lèvre et le capuchon (223 ; 323 ; 423 ; 623) dans une position fermée.

7. Ensemble de fiole selon l'une quelconque des revendications 4 à 6, dans lequel le chemisage souple (441) est surmoulé sur une surface intérieure ou extérieure de la paroi de tube (107 ; 207 ; 307 ; 407 ; 507 ; 607 ; 707).
